# EUROPÄISCHE PATENTSCHRIFT

(11) **EP 1 059 282 B1**
(45) Veröffentlichungstag und Bekanntmachung des Hinweises auf die Patenterteilung: **07.11.2001**
(21) Anmeldenummer: 00108029.0
(22) Anmeldetag: 20.04.2000
(51) Int. Cl.: C07C 67/317, C07C 69/54, C09K 19/20

(54) **Herstellung von acrylierten flüssigkristallinen Verbindungen**
Preparation of acrylated liquid crystal compounds
Préparation de composés acryliques liquides cristallins

(30) Priorität: 27.04.1999 DE 19919153
(43) Veröffentlichungstag der Anmeldung: 13.12.2000
(73) Patentinhaber: Consortium für elektrochemische Industrie GmbH, 81379 München (DE)
(72) Erfinder: Stanjek, Volker, Dr., 81479 München (DE); Schindler, Wolfram, Dr., 82008 Unterhaching (DE); Kammel, Thomas, Dr., 81371 München (DE); Häberle, Norman, Dr., 80689 München (DE)
(74) Vertreter: Fritz, Helmut, Dr.

(56) Entgegenhaltungen:
- WO-A-95/07879
- DE-A- 19 716 822
- DE-C- 646 820
- FR-A- 1 211 945
- DATABASE WPI Section Ch, Week 198704 Derwent Publications Ltd., London, GB; Class A41, AN 1987-027588 XP002143441 & JP 61 286346 A (MITSUI TOATSU CHEM INC), 16. Dezember 1986 (1986-12-16)

## Beschreibung

Die Erfindung betrifft ein einstufiges Verfahren zur Herstellung von Acryloylgruppen enthaltenden, flüssigkristallinen Monomeren.

In der Regel werden für die Erzeugung von dreidimensionalen Netzwerken mit einem gewünschten optisch anisotropen Eigenschaftsprofil vernetzbare flüssigkristalline Monomere benötigt, in denen die mesogenen Einheiten und die vernetzungsfähigen Gruppen durch Spacereinheiten voneinander getrennt sind. Der Einbau der Spacer erhöht dabei die Beweglichkeit der Acrylgruppen und ist zum Erreichen eines hinreichend hohen Vernetzungsgrades unerläßlich. Außerdem kann man durch die Wahl geeigneter Spacerlängen die flüssigkristalline Phase der Monomere positiv beeinflussen.

Auf Grund der Notwendigkeit eines Spacers können die gewünschten Monomere jedoch nicht durch eine einfache (Meth-)acrylierung der meist leicht zugänglichen mesogenen Alkohole erhalten werden. Hier müssen z.T. deutlich aufwendigere Syntheserouten beschritten werden. Dabei ist es in vielen Fällen günstig, wenn der Spacer über eine Etherbindung an die mesogene Einheit gebunden ist. Die entsprechenden Verbindungen sind auf Grund ihrer relativ guten synthetischen Zugänglichkeit, ihrer chemischen Stabilität, vor allem aber wegen ihres oftmals sehr vorteilhaften flüssigkristallinen Eigenschaftsprofils besonders zweckmäßig.

In der Literatur sind daher zahlreiche Darstellungswege beschrieben, die zur Synthese von vernetzungsfähigen Mesogenen mit entsprechenden Spacern prinzipiell geeignet sind. Jedoch sind diese Syntheserouten ausnahmslos mit jeweils spezifischen Nachteilen oder Einschränkungen behaftet. So ist beispielsweise in WO 96/24647 und WO 96/23036 ein Verfahren beschrieben, in dem ein mesogenes Diol zunächst mit einem ω-Halogenalkohol umgesetzt wird. Anschließend wird das dabei erhaltene Zwischenprodukt mit (Meth-)acrylsäurechlorid zum fertigen Produkt verestert. Problematisch bei diesem Verfahren ist zum einen, daß sich die als Zwischenprodukte produzierten Alkohole in den meisten Fällen durch schlechte Löslichkeitseigenschaften auszeichnen, so daß ihre Herstellung, insbesondere aber ihre Isolierung und Reinigung sehr aufwendig sind. Zum anderen sind die als Edukte benötigten ω-Halogenalkohole relativ kostenintensive Verbindungen und oftmals auch nicht im technischen Maßstab erhältlich. Daher ist dieser Syntheseweg für eine industrielle Produktion nur wenig geeignet.

In einem anderen Verfahren, beispielsweise beschrieben in WO 98/47979 und EP 0 648 827, wird von ω-Halogen-(meth-)acryloylalkanen ausgegangen, welche mit einem mesogenen Mono- oder Diol in nur einem Schritt zum jeweils gewünschten Endprodukt umgesetzt werden können. Die arbeitsintensive Gewinnung einer mesogenen Zwischenstufe mit hydroxylierten Spacereinheiten entfällt hier. Nachteilig bei diesem Verfahren ist jedoch die Darstellung der als Edukte benötigten ω-Halogen-(meth-)acryloylalkane, da diese stark zu unerwünschten Polymerisationen neigen und daher ohne Stabilisator kaum zu handhaben sind. Besonders deutlich ausgeprägt ist die Polymerisationstendenz im Falle der Acryloylverbindungen, so daß deren Synthese und Aufreinigung in den allermeisten Fällen nur unter Schwiergkeiten möglich ist. Die Übertragung derartiger Reaktionen in den technischen Maßstab erfordert zudem aufwendige sicherheitstechnische Vorkehrungen.

Schließlich werden in der Literatur auch noch mehrere Herstellungsverfahren beschrieben, die einer gänzlich anderen Strategie folgen. Diesen allen ist jedoch gemein, daß sie ausnahmslos nur durch vielstufige und damit sehr aufwendige Synthesewege realisierbar sind.

Der vorliegenden Erfindung liegt die Aufgabe zugrunde, die Nachteile der bekannten Syntheserouten zu überwinden und ein Verfahren zur Verfügung zu stellen, mit dem vernetzbare Mesogene, die über spacergebundene Acrylgruppen verfügen, auf einfache sowie reaktions- und sicherheitstechnisch unbedenkliche Weise hergestellt werden können.

Gegenstand der Erfindung ist ein einstufiges Verfahren zur Herstellung von Acryloylgruppen enthaltenden, flüssigkristallinen Monomeren der allgemeinen Formel (1)

(Z-Y¹-A²-Y²-)ₘ M (-O-A¹-Acryl)ₙ (1),

bei dem mesogene Alkohole der allgemeinen Formel (2)

(Z-Y¹-A²-Y²-)ₘ M (OH)ₙ (2),

mit Estern der 3-Chlorpropionsäure der allgemeinen Formel (3)

ClPr-A¹-X (3),

unter HCl-Abspaltung umgesetzt werden, wobei
- **Acryl**: Acrylat-Rest,
- **ClPr**: 3-Chlorpropionat-Rest,
- **A**^{**1**}: gleiche oder ungleiche Alkylketten-Spacer mit 2-20 C-Atomen, in welchen die Kohlenstoffkette durch einander nicht benachbarte Sauerstoffatome in Etherfunktionen, Schwefel in Thioetherfunktionen oder durch Iminogruppen unterbrochen sein können,
- **A**^{**2**}: Reste A¹ oder chemische Einfachbindung,
- **M**: mesogene Gruppe,
- **X**: austretende Gruppe,
- **Z**: Alkylreste oder vernetzbare Gruppen
- **Y**^{**1**}**, Y**^{**2**}: unabhängig voneinander chemische Einfachbindung, -O-, -S-, -O-CO-, -CO-O-,-O-CO-O-, -CO-NR¹-, -NR¹-CO-, -O-CO-NR¹-, -NR¹-CO-O- oder -NR¹-CO-NR¹-,
- **R**^{**1**}: Wasserstoff oder C₁-C₄-Alkylrest,
- **n**: Werte 1, 2, 3 oder 4,
- **m**: Werte 0, 1, 2, oder 3 bedeuten.

Bei dem erfindungsgemäßen Verfahren wird von einem Mesogen der allgemeinen Formel (2) mit einer oder mehreren freien Hydroxylgruppen ausgegangen. Die Erfindung beruht dabei auf der Entdeckung, daß man in einem einzigen Syntheseschritt gleichzeitig unter Abspaltung der austretenden Gruppe **X** die Etherbindung zwischen dem mesogenen Alkohol der allgemeinen Formel (2)und dem 3-Chlorpropionsäureester allgemeinen Formel (3) knüpfen und durch Eliminierung eines HCl-Moleküls die Acryloylgruppe aus der Chlorpropionsäureestergruppierung freisetzen kann.

Das erfindungsgemäße Verfahren ist im hohen Maße zur Herstellung von Mesogenen mit ein bis vier spacergebundenen Acrylgruppen, wobei **n** Werte von 1 bis 4, insbesondere 2 aufweist, geeignet. So wird das gewünschte Mesogen der allgemeinen Formel (1)zum einem in nur einem einzigem Syntheseschritt aus gut zugänglichen Edukten dargestellt, zum anderen erlaubt das erfindungsgemäße Verfahren ein Synthesekonzept, in dem die Acrylsäurefunktionen in den eingesetzten Vorstufen, den 3-Chlorpropionsäureestern der allgemeinen Formel (2), maskiert sind. Die Demaskierung erfolgt erst während des letzten Reaktionsschrittes der gesamten Mesogensynthese, der beschriebenen Veretherung eines mesogenen Alkohols. Somit werden in der Synthesesequenz zur Erzeugung acrylierter Mesogene der allgemeinen Formel (1) ausschließlich nicht polymerisierbare Edukte eingesetzt und auch keinerlei polymerisierbare Zwischenstufen gebildet. Die Durchführung der einzelnen Syntheseschritte, vor allem aber die Handhabung der Zwischenprodukte ist damit wesentlich erleichtert. Ein weiterer Vorteil des erfindungsgemäßen Verfahrens liegt in der Tatsache, daß sich die dabei als Edukte benötigten Ester der 3-Chlorpropionsäure der allgemeinen Formel (3) problemlos durch Reaktionen geeigneter Alkohole oder cyclischer Ether mit 3-Chlorpropionsäurechlorid, einer großtechnisch produzierten Chemikalie, darstellen lassen. Auch eine direkte Veresterung der freien 3-Chlorpropionsäure, beispielsweise durch azeotrope Veresterung mit den gesuchten Alkoholderivaten, ist möglich. Dadurch ist die erfindungsgemäße Reaktion auch für industrielle Anwendungen äußerst interessant.

Das erfindungsgemäße Verfahren ist zur Darstellung beliebiger flüssigkristalliner Verbindungen mit einer oder mehreren spacergebundenen Acrylgruppen der allgemeinen Formel (1) geeignet. Bevorzugt eingesetzt wird es für die Darstellung von flüssigkristallinen Bisacrylaten der allgemeinen Formel (1), in der **m** = 0, **n** = 2 sind, durch die Umsetzung eines mesogenen Diols der allgemeinen Formel (2), in der **m** = 0, **n** = 2 sind, mit 2 Äquivalenten einer der allgemeinen Formel (3) entsprechenden Verbindung. Durch den Einsatz von Gemischen mehrerer der allgemeinen Formel (3) entsprechenden Verbindungen lassen sich dabei auch stöchiometrische Mischungen aus symmetrischen und asymmetrischen Bisacrylaten erzeugen. Bevorzugt wird nur eine Verbindung der allgemeinen Formel (3) eingesetzt.

Ferner kann man durch das erfindungsgemäße Verfahren auch flüssigkristalline Alkohole umsetzten, welche bereits über vernetzungsfähige Gruppen der allgemeinen Formel (2) verfügen, in der **m** > 0, **Z** = vernetzungsfähige Gruppen bedeuten. Dies kann man auch zur Darstellung von Mesogenen mit zwei unterschiedlichen vernetzbaren Gruppen und/oder Spacern ausnutzen, wobei dann für die allgemeinen Formeln (1) und (2) gilt: **m** = 1, **n** = 1 und **Z** = vernetzungsfähige Gruppe.

Als Reste **M** kommen alle bekannten mesogenen Gruppen in Betracht. Bevorzugt entspricht **M** der allgemeinen Formel (4)

(-T-Y³)ᵣ-T- (4),

in der
- **T**: zweiwertige gesättigte oder ungesättigte iso- oder heterocyclische, gegebenenfalls substituierte Kohlenwasserstoffreste mit 5-20 C-Atomen, wobei Benzyl-, Cyclohexyl- und Naphthylreste bevorzugt sind,
- **Y**^{**3**}: chemische Einfachbindung, -O-, -S-, -O-CO-, -CO-C-, -O-CO-O-, -CO-NR²-, -NR²-CO-, -O-CO-NR²-, -NR²-CO-O-, -NR²-CO-NR²-, -CH₂-O-, -O-CH₂-, -CH=N-, -N=CH- oder -N=N-,
- **R**^{**2**}: Wasserstoff oder C₁-C₄-Alkylrest,
- **r**: Werte 0, 1, 2, 3 oder 4, wobei 1 oder 2 besonders bevorzugt sind, bedeuten.

Im Falle von **r** > 0 können die Reste **T** wie auch die verbrückenden Reste **Y**^{**3**} jeweils untereinander identisch oder auch unterschiedlich sein. Die Reste **T** können dabei auch höher substituiert sein, z.B. durch C₁-C₄-Alkylgruppen, Fluor, Chlor, Brom, Cyano-, Hydroxy-, (Alkyl-)Amino-, oder auch Nitrogruppen. Besonders bevorzugte Reste **T** sind Benzyl- oder Cyclohexylgruppen, welche gegebenenfalls weitere Substituenten tragen.

Besonders bevorzugt sind mesogene Gruppen **M**, die den Strukturformeln (5) oder (6) 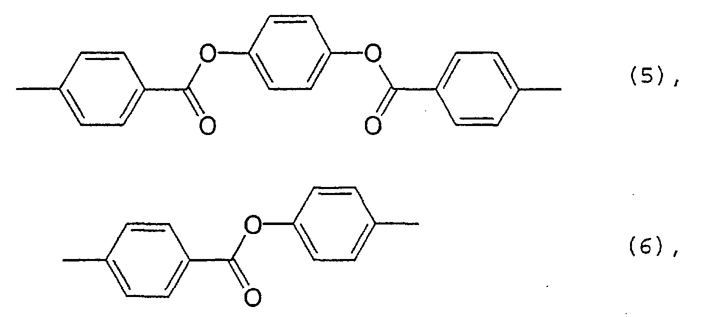 entsprechen, wobei jeder Ring einen oder mehrere identische oder unterschiedliche Substituenten tragen kann.

Besonders bevorzugte mesogene Diole der allgemeinen Formel (1) entsprechen den allgemeinen Formeln (7) bzw. (8)

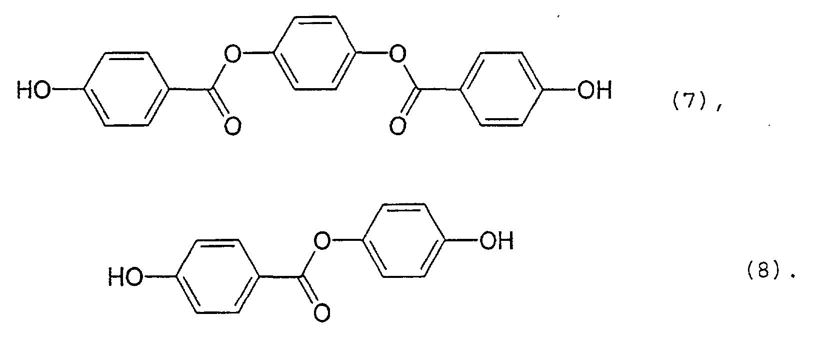

Diese sind über bekannte Synthesewege problemlos aus Hydrochinon und *p*-Hydroxybenzoesäure bzw. aus substituierten Derivaten dieser Verbindungen zugänglich, wie beispielsweise in *Polym. Prep.* 30 (2), 462-3, *1989 beschrieben.*

Die Alkylreste **Z** in den allgemeinen Formeln (1) und (2) sind vorzugsweise C₁-C₄-Alkylreste. Als vernetzbare Gruppen **Z** können alle dem Fachmann bekannten Gruppen fungieren. Bevorzugt als Gruppen b sind Alkenylgruppen, wie Vinyl- 2-Methylvinyl- und Allylgruppen; Alkinylgruppen oder Epoxygruppen.

Als Abgangsgruppe **X** in der allgemeinen Formel (3) können alle dem Fachmann bekannten Gruppen fungieren. Bevorzugt werden Halogene oder Sulfonsäurederivate, wobei letztere, wie z.B. Triflat der Formel (9), auch über fluorhaltige Reste verfügen können. Die Formeln (9) - (13) 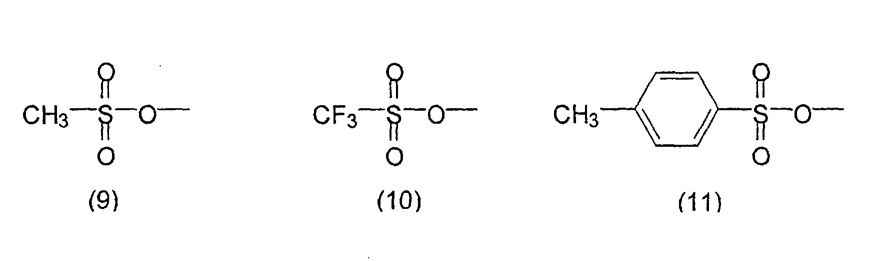 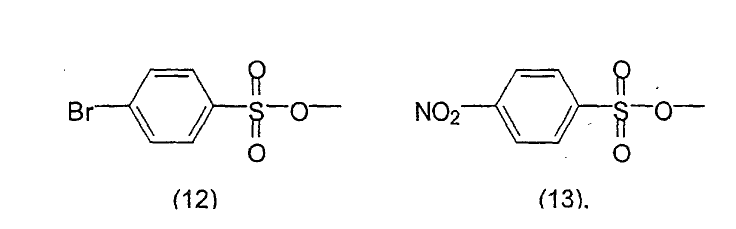 stellen Beispiele für derartige bevorzugte Sulfonsäurereste dar.
Als Abgangsgruppen **X** besonders bevorzugt sind Chlor- oder Brom-Abgangsgruppen, bei es von Vorteil ist, die erfindungsgemäße Veretherung in Gegenwart katalytischer Mengen Kaliumjodid durchzuführen.

Zur Darstellung von geeigneten Verbindungen der allgemeinen Formel (3) steht dem Chemiker ein äußerst umfangreiches Repertoire unterschiedlicher Verfahren zur Verfügung. Bei der formal einfachsten Reaktion wird von einem ω-Halogenalkohol ausgegangen, welcher mit 3-Chlorpropionsäure-chlorid zum entsprechenden Ester umgesetzt wird (X = Halogen). Auf Grund der beschränkten Verfügbarkeit preiswerter ω-Halogenalkohole ist dieser Weg jedoch nur in Ausnahmefällen technisch interessant.
Hier ist es in der Regel günstiger, von beliebigen Alkyldiolen auszugehen. Diese können beispielsweise unter geeigneten Bedingungen mit 3-Chlorpropionsäurechlorid zu den entsprechenden 3-Chlorpropionsäuremonoestern umgesetzt werden. Aus diesen lassen sich anschließend durch Veresterung der noch freien Hydroxylfunktion mit Sulfonsäurechloriden problemlos aktivierte Sulfonsäurederivate der allgemeinen Formel (3) herstellen, in der **X** ein Sulfonsäurederivat bedeutet.

Des weiteren ist es auch möglich, die freie Hydroxylgruppe des erwähnten 3-Chlorpropionsäuremonoesters gegen Halogenatome auszutauschen, z.B. durch eine Reaktion mit Thionylchlorid. Auch die dadurch darstellbaren Verbindungen sind als Edukt der allgemeinen Formel (3), in der **X** ein Halogenatom bedeutet, geeignet.

Eine besonders effiziente sowie elegante Reaktion zur Herstellung einer Verbindung der allgemeinen Formel (3)ist die Ringöffnung cyclischer Ether wie Tetrahydrofuran durch 3-Chlorpropionsäurechlorid. Hier wird 3-Chlorpropionsäure-4-chlorbutylester in nur einem einzigen, sehr einfachen Reaktionsschritt aus äußerst preisgünstigen Edukten hergestellt.

In den folgenden Beispielen sind, falls jeweils nicht anders angegeben, alle Mengen- und Prozentangaben auf das Gewicht bezogen, alle Drücke 0,10 MPa (abs.) und alle Temperaturen 20°C.

### Beispiel 1: Darstellung von 4-Chlorbutyl-3-chlorpropionat

89,4 ml (114,2 g, 0,9 mol) 3-Chorpropionsäurechlorid werden in 160 ml Cyclohexan gelöst. Man gibt 613 mg (4,5 mmol) Zinkchlorid hinzu und überlagert die erhaltene Suspension mit Stickstoff. Anschließend erhitzt man bis zum Sieden und tropft langsam und unter gutem Rühren 80,4 ml (71,4 g, 0,99 mol) THF hinzu. Die Reaktion verläuft stark exotherm - die Zugabegeschwindigkeit des THF ist daher so zu regeln, daß die Reaktionsmischung auch ohne äußere Wärmezufuhr gleichmäßig siedet. Nach der vollständigen Zugabe des THF wird für weitere 30 min unter Rückfluß gerührt.
Zur Aufarbeitung läßt man auf Raumtemperatur abkühlen und wäscht die Reaktionsmischung mit 80 ml NaOH-Lösung (5 %-ig) und zweimal mit 80 ml destilliertem Wasser. Bei 60 °C im Teilvakuum werden anschließend Cyclohexan und Restwasser entfernt und das Produkt wird durch Destillation (84 °C bei 1 mbar) gereinigt. Ausbeute: 164,6 g (92 % der Theorie).

### Beispiel 2: Darstellung von Hydrochinon-bis-[4-(4-acryloyloxybutoxy)-benzoat]

292 g (2,1 mol) Kaliumcarbonat, 5 g (30 mmol) Kaliumjodid und 0,4 g (1,5 mmol) 2,6-Di-t-butyl-4-(N,N-dimethylaminomethyl)-phenol als Stabilisator werden in 250 ml N-Methyl-2-pyrrolidinon suspendiert. Anschließend gibt man unter gutem Rühren 105,1 g (0,30 mol) Hydrochinon-bis-(4-hydroxybenzoat) hinzu. Nach dem Erreichen der Reaktionstemperatur von 100 °C werden 144,0 g (0,722 mol) 4-Chlorbutyl-3-chlorpropionat hinzugetropft. Schließlich rührt man für weitere 4 h bei dieser Temperatur.
Zur Aufarbeitung wird auf 80 °C abgekühlt und man gibt 300 ml Xylol hinzu. Bei dieser Temperatur wäscht man einmal mit 400 ml Wasser, einmal mit 250 ml Wasser und noch zweimal mit 50 ml Wasser. Anschließend wird die organische Phase durch azeotrope Destilliation von ca. 50 ml Xylol im Teilvakuum getrocknet. Beim Abkühlen auf 15 °C kristallisiert das saubere Produkt aus. Ausbeute: 135,5 g (75 % der Theorie).

### Beispiel 3: Darstellung von 4-(4-Acryloyloxybutoxy)-phenol-[4-(4-acryloyloxybutoxy)-benzoat]

110,4 g (0,8 mol) Kaliumcarbonat, 1,7 g (10 mmol) Kaliumjodid und 0,2 g (0,7 mmol) 2,6-Di-t-butyl-4-(N,N-dimethylaminomethyl)-phenol als Stabilisator werden in 120 ml N-Methyl-2-pyrrolidinon suspendiert. Anschließend gibt man unter gutem Rühren 23,0 g (0,10 mol) Hydrochinon-mono-(4-hydroxybenzoat) hinzu. Nach dem Erreichen der Reaktionstemperatur von 100 °C werden 59,7 g (0,3 mol) 4-Chlorbutyl-3-chlorpropionat hinzugetropft. Schließlich rührt man für weitere 4 h bei dieser Temperatur.
Zur Aufarbeitung wird auf 50 °C abgekühlt und man gibt 200 ml Xylol hinzu. Bei dieser Temperatur wäscht man einmal mit 300 ml Wasser, einmal mit 150 ml Wasser und noch zweimal mit 25 ml Wasser. Anschließend engt man die organische Phase im Vakuum ein und kristallisiert das erhaltene Rohprodukt aus Isopropanol (50 ml) um.
Ausbeute: 37,6 g (78 % der Theorie).

### Beispiel 4: Darstellung von 3-Chlorpropyl-3-chlorpropionat

101,5 ml (129,6 g, 1,00 mol) 3-Chlorpropionylchlorid werden bei 90 °C zu 86,1 ml 3-Chlor-1-propanol (97,4 g, 1,00 mol) getropft (Dauer ca. 30 min). Während der Zugabe kommt es zu einer spontanen Gasentwicklung und die Reaktionstemperatur fällt auf ca. 70°C. Nach Beendigung der Zugabe wird die Reaktionsmischung erneut auf 100 °C erhitzt und für weitere 30 min bei dieser Temperatur gerührt. Nach dem Abkühlen wird das erhaltene Rohprodukt destillativ gereinigt.
Ausbeute: 186,4 g (98 % der Theorie).

### Beispiel 5: Darstellung von Hydrochinon-bis-[4-(3-acryloyloxypropoxy)-benzoat] aus 3-Chlorpropyl-3-chlorpropionat

193,2 g (1,40 mol) Kaliumcarbonat, 8,3 g (0,05 mol) Kaliumjodid und 0,3 g 2,6-Di-t-butyl-4-(N,N-dimethylaminomethyl)-phenol als Stabilisator werden in 230 ml in N-Methyl-2-pyrrolidinon suspendiert und auf 60°C aufgeheizt. Bei dieser Temperatur werden 70,1 g (0,20 mol) Hydrochinon-bis-(4-hydroxybenzoat) zugegeben. Anschließend wird weiter auf 95°C erwärmt. Nach dem Erreichen dieser Temperaur werden 92,6 g (0.48 mol) 3-Chlorpropyl-3-chlorpropionat binnen 15 min zugetropft. Danach wird für weitere 5h bei 95 °C gerührt.
Zur Aufarbeitung wird auf 80 °C abgekühlt und man gibt 200 ml Xylol hinzu. Bei dieser Temperatur wäscht man einmal mit 270 ml Wasser, einmal mit 180 ml Wasser und noch zweimal mit 40 ml Wasser. Anschließend wird die organische Phase durch azeotrope Destillation von ca. 50 ml Xylol im Teilvakuum getrocknet. Beim Abkühlen auf 15 °C kristallisiert das saubere Produkt aus. Dieses wird abfiltriert und mit EtOH gewaschen.
Ausbeute: 76,0 g (62% der Theorie).

### Beispiel 6: Darstellung von (4-Acryloyloxybutoxy)-phenol-(4-allyloxybenzoat) aus 4-Chlorbutyl-3-chlorpropionat

162,1 g (1,0 mol) Kaliumcarbonat, 3,3 g (20 mmol) Kaliumjodid und 0,2 g (0,8 mmol) 2,6-Di-t-butyl-4-(N,N-dimethylaminomethyl)-phenol als Stabilisator werden in 150 ml N-Methyl-2-pyrrolidinon suspendiert. Anschließend gibt man unter guter Rührung 54,0 g (0,2 mol) 4-Allyloxybenzoesäurehydrochinonester hinzu. Nach dem Erreichen der Reaktionstemperatur von 100 °C werden 79,6 g (0,4 mol) 4-Chlorbutyl-3-chlorpropionat hinzugetropft. Schließlich rührt man für weitere 4 h bei dieser Temperatur.
Zur Aufarbeitung wird auf 80 °C abgekühlt und man gibt 110 ml Xylol hinzu. Bei dieser Temperatur wäscht man einmal mit 300 ml Wasser, einmal mit 200 ml Wasser, einmal mit 100 ml Wasser, einmal mit 100 ml 10 %iger Natronlauge und weitere zweimal mit 150 ml Wasser. Anschließend wird die organische Phase durch azeotrope Destillation von 30 ml Xylol im Teilvakuum getrocknet. Der dabei als Nebenprodukt entstandene Bis-4-Allyloxybenzoesäurehydrochinonester wird nach Abkühlung auf 50 °C abfiltriert. Nach der Zugabe von 100 ml Ethanol kristallisiert das Produkt bei 0 °C aus.
Ausbeute: 32,9 g (38 % der Theorie).

## Patentansprüche

1. Einstufiges Verfahren zur Herstellung von Acryloylgruppen enthaltenden, flüssigkristallinen Monomeren der allgemeinen Formel (1)
(Z-Y¹-A²-Y²-)ₘ M (-O-A¹-Acryl)ₙ (1),
bei dem mesogene Alkohole der allgemeinen Formel (2)
(Z-Y¹-A²-Y²-)ₘ M (OH)ₙ (2),
mit Estern der 3-Chlorpropionsäure allgemeinen Formel (3)
ClPr-A¹-X (3),
unter HCl-Abspaltung umgesetzt werden, wobei
**Acryl** Acrylat-Rest,
**ClPr** 3-Chlorpropionat-Rest,
**A**^{**1**} gleiche oder ungleiche Alkylketten-Spacer mit 2-20 C-Atomen, in welchen die Kohlenstoffkette durch einander nicht benachbarte Sauerstoffatome in Etherfunktionen, Schwefel in Thioetherfunktionen oder durch Iminogruppen unterbrochen sein können,
**A**^{**2**} Reste **A**^{**1**} oder chemische Einfachbindung,
**M** mesogene Gruppe,
**X** austretende Gruppe,
**Z** Alkylreste oder vernetzbare Gruppen
**Y**^{**1**}**, Y**^{**2**} unabhängig voneinander chemische Einfachbindung, -O-, -S-, -O-CO-, -CO-O-,-O-CO-O-, -CO-NR¹-, -NR¹-CO-, -O-CO-NR¹-, -NR¹-CO-O- oder -NR¹-CO-NR¹-,
**R**^{**1**} Wasserstoff oder C₁-C₄-Alkylrest,
**n** Werte 1, 2, 3 oder 4,
**m** Werte 0, 1, 2, oder 3 bedeuten.

2. Verfahren nach Anspruch 1, bei dem **n** den Wert 2 aufweist.

3. Verfahren nach Anspruch 1 oder 2, bei dem **m** den Wert 0 aufweist.

4. Verfahren nach Ansprüchen 1 bis 3, bei dem **M** der allgemeinen Formel (4)
(-T-Y³)ᵣ-T- (4),
entspricht, in der
**T** zweiwertige gesättigte oder ungesättigte iso- oder heterocyclische gegebenenfalls substituierte Kohlenwasserstoffreste mit 5-20 C-Atomen,
**Y**^{**3**} chemische Einfachbindung, -O-, -S-, -O-CO-, -CO-O-, -O-CO-O-, -CO-NR²-, -NR²-CO-, -O-CO-NR²-, -NR²-CO-O-, -NR²-CO-NR²-, -CH₂-O-, -O-CH₂-, -CH=N-, -N=CH- oder -N=N-,
**R**^{**2**} Wasserstoff oder C₁-C₄-Alkylrest und
**r** Werte 0, 1, 2, 3 oder 4, bedeuten.

5. Verfahren nach Anspruch 1 bis 4, bei dem die mesogenen Gruppen **M** den Strukturformeln (5) oder (6) 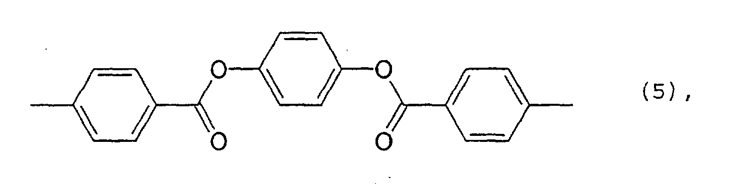 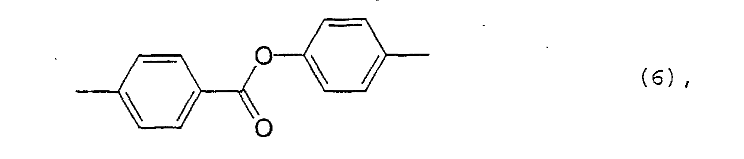 entsprechen, wobei jeder Ring Substituenten tragen kann.

6. Verfahren nach Anspruch 1 bis 5, bei dem nur eine Verbindung der allgemeinen Formel (3) eingesetzt wird.

7. Verfahren nach Anspruch 1 bis 6, bei dem die Gruppe **X** Halogen oder Sulfonsäurederivat bedeutet.

## Claims

1. One-step process for the preparation of acryloyl group-containing liquid-crystalline monomers of the general formula (1)
(Z-Y¹-A²-Y²-)ₘ M (-O-A¹-acrylate)ₙ (1)
in which mesogenic alcohols of the general formula (2)
(Z-Y¹-A²-Y²-)ₘ M (OH)ₙ (2)
are reacted with esters of 3-chloropropionic acid of the general formula (3)
ClPr-A¹-X (3)
with elimination of HCl, where
**acrylate** is an acrylate radical,
**ClPr** is a 3-chloropropionate radical,
**A**^{**1**} are identical or different alkyl chain spacers having 2-20 carbon atoms, in which the carbon chain may be interrupted by non-adjacent ether oxygen atoms, thioether sulphur or imino groups,
**A**^{**2**} are radicals **A**^{**1**} or single chemical bonds,
**M** is a mesogenic group,
**X** is a leaving group,
**Z** are alkyl radicals or crosslinkable groups,
**Y**^{**1**} **and Y**^{**2**}**,** independently of one another, are a single chemical bond, -O-, -S-, -O-CO-, -CO-O-, -O-CO-O, -CO-NR¹-, -NR¹-CO-, -O-CO-NR¹-, -NR¹-CO-O- or -NR¹-CO-NR¹-,
**R**^{**1**} is hydrogen or a C₁-C₄-alkyl radical,
**n** has a value of 1, 2, 3 or 4, and
**m** has a value of 0, 1, 2 or 3.

2. Process according to Claim 1, in which n has the value 2.

3. Process according to Claim 1 or 2, in which m has the value 0.

4. Process according to Claims 1 to 3, in which M conforms to the general formula (4)
(-T-Y³)ᵣ-T- (4)
in which
**T** are divalent saturated or unsaturated, isocyclic or heterocyclic, substituted or unsubstituted hydrocarbon radicals having 5-20 carbon atoms,
**Y**^{**3**} is a single chemical bond, -O-, -S-, -O-CO-, -CO-O-, -O-CO-O-, -CO-NR²-, -NR²-CO-, -O-CO-NR²-, -NR²-CO-O-, -NR²-CO-NR²-, -CH₂-O-, -O-CH₂-, -CH=N-, -N=CH- or -N=N-,
**R**^{**2**} is hydrogen or a C₁-C₄-alkyl radical, and
**r** has a value of 0, 1, 2, 3 or 4.

5. Process according to Claims 1 to 4, in which the mesogenic groups **M** conform to the structural formula (5) or (6) 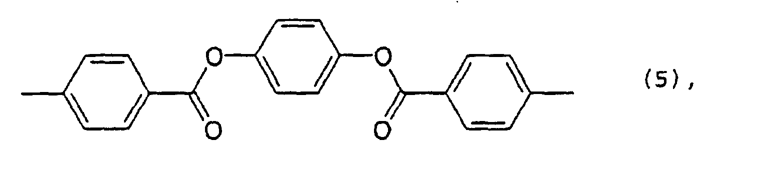 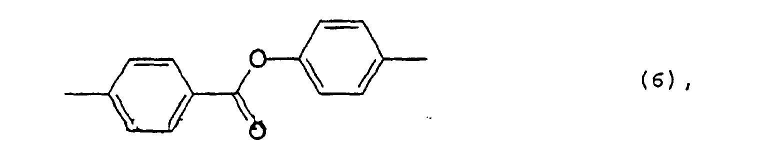 where each ring can carry substituents.

6. Process according to Claims 1 to 5, in which only one compound of the general formula (3) is employed.

7. Process according to Claims 1 to 6, in which the group **X** is halogen or a sulphonic acid derivative.

## Revendications

1. Procédé en une étape pour la préparation de monomères de type cristal liquide contenant des groupes acryloyle, de formule générale (1)
(Z-Y¹-A²-Y²-)ₘ M (-O-A¹-acryl)ₙ (1),
dans lequel on fait réagir des alcools mésogènes de formule générale (2)
(Z-Y¹-A²-Y²-)ₘ M (OH)ₙ (2)
avec des esters de l'acide 3-chloropropionique de formule générale (3)
ClPr-A¹-X (3)
avec élimination de HCl,
formules dans lesquelles
acryl représente le radical acrylate,
ClPr représente le radical 3-chloropropionate,
A¹ représente des espaceurs constitués de chaînes alkyle identiques ou différentes ayant de 2 à 20 atomes de carbone, dans lesquels les chaînes hydrocarbonées peuvent être interrompues par des atomes d'oxygène en fonctions éther, des atomes de soufre en fonction thioéther, non adjacents les uns aux autres, ou par des groupes imino,
A² représente des radicaux A¹ ou une simple liaison chimique,
M représente un groupe mésogène,
X représente un groupe partant,
Z représente des radicaux alkyle ou des groupes réticulables,
Y¹, Y² représentent, indépendamment l'un de l'autre, une simple liaison chimique, -O-, -S-, -O-CO-, -CO-O-, -O-CO-O-, -CO-NR¹-, -NR¹-CO-, -O-CO-NR¹-, -NR¹-CO-O- ou -NR¹-CO-NR¹-,
R¹ représente un atome d'hydrogène ou un radical alkyle en C₁-C₄,
n a les valeurs 1, 2, 3 ou 4,
m a les valeurs 0, 1, 2 ou 3.

2. Procédé selon la revendication 1, dans lequel n a la valeur 2.

3. Procédé selon la revendication 1 ou 2, dans lequel m a la valeur 0.

4. Procédé selon les revendications 1 à 3, dans lequel M correspond à la formule générale (4)
(-T-Y³)ᵣ-T- (4)
dans laquelle
T représente des radicaux hydrocarbonés iso- ou hétérocycliques bivalents saturés ou insaturés, éventuellement substitués, ayant de 5 à 20 atomes de carbone,
Y³ représente une simple liaison chimique, -O-, -S-, -O-CO-, -CO-O-, -O-CO-O-, -CO-NR²-, -NR²-CO-, -O-CO-NR²-, -NR²-CO-O-, -NR²-CO-NR²-, -CH₂-O-, -O-CH₂-, -CH=N-, -N=CH- ou -N=N-,
R² représente un atome d'hydrogène ou un radical alkyle en C₁-C₄ et
r a les valeurs 0, 1, 2, 3 ou 4.

5. Procédé selon les revendications 1 à 4, dans lequel les groupes mésogènes M correspondent aux formules développées (5) ou (6) 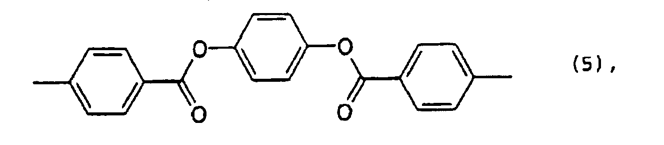 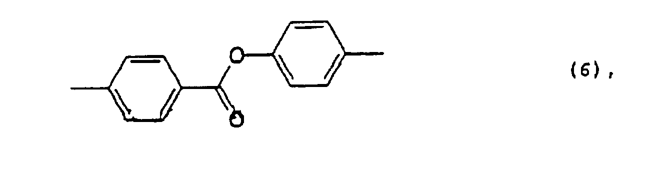 chaque cycle pouvant porter des substituants.

6. Procédé selon les revendications 1 à 5, dans lequel on utilise qu'un seul composé de formule générale (3).

7. Procédé selon les revendications 1 à 6, dans lequel le groupe X représente un atome d'halogène ou un dérivé d'acide sulfonique.
